# EUROPEAN PATENT SPECIFICATION

(11) **EP 1 980 546 B1**
(45) Date of publication and mention of the grant of the patent: **29.03.2017**
(21) Application number: 08100827.8
(22) Date of filing: 23.01.2008
(51) Int. Cl.: C05F 17/00, C05F 17/02

(54) **Process and system for the production of energy and composted material from agricultural waste containing cellulose**
Verfahren und System zur Herstellung von Energie und kompostiertes Material aus Landwirtschaftsabfällen mit Zellulose
Procédé et système pour la production d'énergie et de matériau composté à partir de déchets agricoles contenant de la cellulose

(30) Priority: 26.01.2007 IT MI2007 116
(43) Date of publication of application: 15.10.2008
(73) Proprietor: Neorurale S.p.A., 20124 Milano (IT)
(72) Inventor: Natta, Giuseppe, 27010 Giussago (PV) (IT); Garuti, Gilberto, 27010 Giussago (PV) (IT); Natta, Francesco, 27010 Giussago (PV) (IT)
(74) Representative: Minoja, Fabrizio

(56) References cited:
- EP-A1- 0 755 905
- EP-A2- 0 934 998
- WO-A1-01/05729
- WO-A2-2006/120517
- DE-A1- 19 719 323
- US-A1- 2004 191 755

## Description

### FIELD OF THE INVENTION

The present invention relates in general to a process and system whereby agricultural by-products (straw, pruning waste, etc.) that are rich in cellulose are treated biologically by an anaerobic and aerobic process.

More particularly the process that forms the object of the present invention sets out to solve the problem of the use of rice straw for which specific provision has not at present been made.

The production of rice straw is set at around 5 tonnes per hectare and per year and in a rice growing area such as Lombardy and Piedmont around 210,000 hectares are cultivated.

The annual production of straw in said regions can therefore be estimated at 1,000,000 tonnes which, compressed into bales with density of 200 kg/m3, produces a total volume of 5 million m³.

Given the lack of other solutions, the straw is normally incorporated into the land by ploughing or deep harrowing.

In anoxic land such as rice fields, the straw undergoes anaerobic degradation and is transformed into methane which is released into the atmosphere.

Although on the one hand it is true that the carbon released into the atmosphere is biological in origin and therefore renewable, on the other hand it is known that methane has a greenhouse effect which is 21 times that of CO₂.

It is estimated that the methane released in a year is around 10 tonnes CO₂ equivalent per hectare (Holzapfel-Pschorn and Seiler, 1986) and therefore, for the area of 210,000 hectares considered, of the order of magnitude of 2.1 million tonnes, a quantity of CO₂ of the order of magnitude of that emitted by a thermoelectric power plant with power of 560 MW in the territory.

Moreover the procedure of burying straw may encourage the development of stem rot disease (*Sclerotium oryzae*) (Bockus et al., 1979) which farmers seek to remedy by burning straw directly in the field, creating strong environmental impact above all due to the fine dust emitted in the area (Jenkins, 1996). In certain states such as for example California this process is banned, forcing the recycling of biomasses (Kadam et al., 2000).

The present invention therefore refers to the agricultural industry, yet involves strong environmental effects on the territory and, as will be made clearer herein below, may have considerable impact on the energy policy of entire regions.

### STATE OF THE ART

To solve the above environmental problems and transform biomasses into energy the techniques recently proposed are combustion and anaerobic digestion to produce biogas as energy carrier.

Combustion with production of steam for the generation of electrical energy is however a capital intensive operation which is poorly suited to a group of farms.

It is widely used in the case of industrial waste and municipal solid waste where there is a tariff for the collection of the same waste which allows the high industrial costs to be offset.

Combustion in small plants for producing thermal energy is impractical due to the difficulty of storage, feeding of the straw and its combustion in simple systems, to the extent that in the past practical energy uses have never been found, unlike wood.

Anaerobic digestion for producing biogas is instead more compatible with the economy and practice of rural firms which for years have carried out, in small local plants, digestion of liquid manure from stables to produce a part of the electrical energy used on the farms.

For this reason most patents for the treatment of solid waste and more particularly straw propose fine shredding of this farming waste and its mixing with other effluent in liquid suspension.

The patent EP1456343, for example, proposes the use of flotation tanks wherein the solids are held in suspension by the gas generated, EP0822251 a compartmentalised horizontal tank, EP0520172 a pulping system operating continuously followed by digestion reactors provided with a stirrer, EP0142873 two reaction stages, the first hydrolysis wherein the coarser material is retained and the second digestion, GB2144767 large horizontal tanks operating on a semibatch basis and provided with vertical worm stirrers, US2006102560 a system operating with continuous reactor and in slurry, DE102004031170 large spherical tanks and WO111124 the use of an abandoned mine where solid and liquid waste is fed in the form of slurry and biogas extracted.

All these patents, as already mentioned, appear as an extension of the small systems for treating farm liquid manure and slurry and some problems are tackled such as the large volume and quantity of the solids to be treated as indicated by the last patent mentioned which even uses a mine.

Another problem can relate to the lower reactivity of the solids compared to the liquid manure or slurry which requires high storage times and therefore once again high volumes and which rules against the use of systems operating continuously.

For this reason there is no knowledge of applications on a large scale of processes treating solid agricultural waste and in particular straw in suspension in the liquid phase, while methods which operate in the dry phase and discontinuously are gaining ground.

Among these mention can be made of DE10302658 which operates on a pile basis and which uses a gas lining for heating the solids to the temperature useful for mesophilic digestion, EP0023176, CA2533213, EP1428868 which use a kind of box or container with a waterproof membrane and an airtight door for the feeding and extraction of solids and a system for feeding water and wall heating, EP1589095 which provides a compartmentalised masonry building for storage, grinding and digestion similar to those used for composting waste, and WO2004004937 a large cement silo with systems for heating on the walls and inside the bed of solids.

The patent application WO2005/054423 provides a series of large modular tunnels wherein the piled waste is placed and extracted via an airtight door. The tunnel is lined with a waterproof membrane, the floor and the walls are heated, the percolate is re-circulated from the base and distributed via sprinklers in the ceiling, free of the waste, onto the pile and biogas is extracted for various thermal uses or for the production of electrical energy.

The patent application, like others in the same series, has been realised industrially by BEKON Technologies GmbH & Co.

US 2004/191755 A1 discloses a method and system for treating municipal solid waste consisting in excavating a cell and stockpile soil for use as a cover, as in common landfills.

EP 0755905 A1 discloses a plant for treating the humid fraction of swill comprising cells made of concrete, metal, wood or other material, placed side by side such as to have a common wall and provided with a system of heating tubes.

WO 2006/120517 A2 discloses a plant consisting of a flexible tubular container provided with one or more rigid end walls for facilitating the transport and introduction of organic waste. In the container, which is sealed during operation through an external wrapping membrane, are inserted tubes for recycling the percolate, for extracting the biogas and for blowing air.

WO 01/05729 A1 discloses an organic waste treatment process using a plant comprising one or more sealed containers made of rigid material such as steel or concrete, wherein the organic waste is fed by means a belt feeder and from which, after treatment, the compost material is extracted.

DE 19719323 A1 discloses a sealed double wall tunnel fermenter made of steel, concrete or rigid plastic, provided with a sealed door for the introduction and extraction of the material, and the insertion of all means for recycling the percolate, blowing air, extraction of biogas and floor heating.

EP 0934998 A2 discloses a system for the aerobic and anaerobic digestion of agricultural waste stored in permeable bags. As an example a single bale is wrapped in a membrane and is punched with a special lance which allows introduction of air, flooding of the bale with water , extraction of biogas and possible feed of heat.

There are also patents applied to dumps of previously dried municipal solid waste which are definitely simpler to apply and which treat significantly higher volumes than all the technologies mentioned hitherto.

Mention can be made for example of EP1520634 of ECODECO (an affiliate of the Applicant) in which municipal solid waste (MSW), after undergoing a process of biodrying via aerobic digestion in a special system, is stored in a bioreactor where it is activated by wetting with water or other liquid waste to produce biogas. After purification the biogas is used in internal combustion engines to produce electrical energy.

With this technology an industrial plant was built by ECODECO at Corteolona (PV) with production of 4.8 MW of electrical energy.

The teaching which is gained from examining the prior art mentioned and from numerous other technologies, not mentioned for the sake of brevity, is the following:
- the wet processes have limited capacities.
- The dry processes reported in the prior art can also be extended via modular systems with higher capacities, dimensioned according to the available capacity of the farm, yet require the building of storage silos or buildings.
- In all dry processes recycling of the percolate as a carrier of digestion microorganisms, in some cases added as inoculum at the start, is important.
- It is also important to maintain the temperature between 30 and 40°C for the purposes of efficacy of mesophilic anaerobic digestion and in this respect small or medium-sized systems with high exchange surfaces involve heating of the walls and of the base of the digesters.
- In systems with the dimensions of an municipal solid waste dump, as in EP1520634, heating can be limited to the initial stage, for example by means of hot water, and the temperature maintained with the heat of the recycling pump and the slight exothermy of the anaerobic digestion. In all the known processes for the production of biogas from biomasses or agricultural waste heating of the mass is carried out through heat coming from other sources or yielded via exchange surfaces.
- It is also important to note in EP1520634 that the presence of an aerobic treatment makes the waste for anaerobic digestion more active, shortening the times of exhaustion of the bioreactor and increasing hourly production of biogas, in addition to transforming waste into dry material which does not produce biogas except after activation with water.

The Applicant produces independently and via associate companies in the provinces of Pavia, Milan and Novara approximately 5,000 tonnes/year of rice and the same number of straw on a surface of approximately 1,000 hectares.

The Applicant is also ideally placed, via the consortium of producers of rice and of other cereals, to extend the application of the object of the present invention to entire areas of Piedmont and Lombardy with much higher production of straw for disposal than the million tonnes mentioned for rice alone.

In these conditions, and as will be described in greater detail herein below, the technologies proposed in the prior art for disposal of agricultural waste appear limitative and difficult to realise in practice.

As will be seen, the benefit in terms of energy contribution and reuse of the farming waste material is high.

### DESCRIPTION OF THE INVENTION

The main object of the invention is to provide an aerobic-anaerobic treatment method for agricultural waste containing cellulose, with particular reference to the straw of cereals and in particular of rice, which allows elimination of the environmental problems caused by the common practice of burying or combustion of the same on the fields.

An integral part of the above object is the total elimination of the olfactory environmental impact arising from the pile of digestible material normally used for the production of biogas.

A further object is that of providing a treatment method which allows recovery of the energy contained in said straw in the form of biogas, to generate thermal and/or electrical energy from renewable sources.

A particular and equally important object is that of providing an economical method for possible application on a farm and on a large scale such on the one hand as to allow systematic disposal of all the agricultural waste present on the territory and on the other hand to provide a significant contribution to the energy requirement of the farms and the territory which houses them.

A further object relates to the processing of the material into a solid which can no longer be digested and which is rich in nutrients to be used as a fertiliser in farming.

The above objects, including therein the absence of olfactory impact, are achieved by the process according to the invention which has the features of the accompanying independent claim 1 and the system of the independent claim 8.

Advantageous embodiments of the invention are disclosed in the dependent claims.

Further features of the invention will be made clearer by the following detailed description, referring to the embodiment given purely by way of a non-limiting example and illustrated in the accompanying drawings, in which:
- Fig. 1 is a schematic view in vertical section of a rural bioreactor according to the present invention;
- Fig. 2 is a plan view of the platform of the bioreactor whereon a comb collector is placed for collecting the percolate and the biogas.

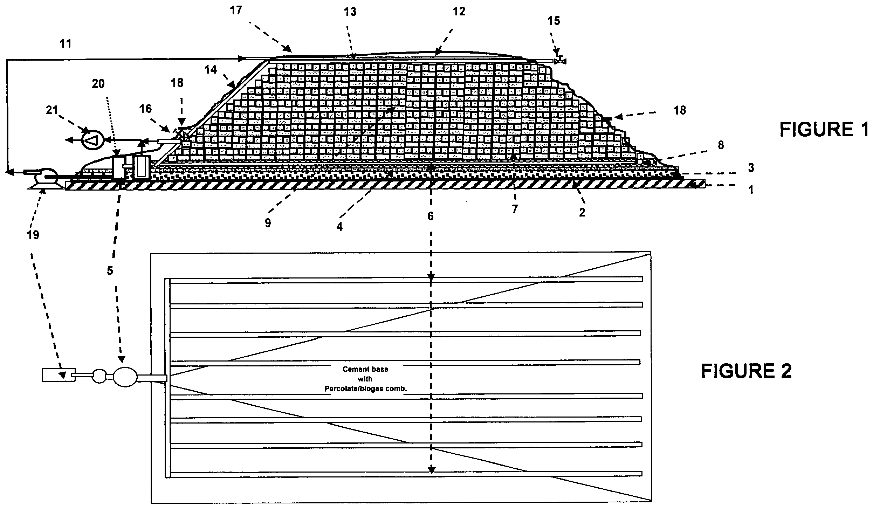

Reference is made to Fig. 1, which shows one of the possible non-limiting embodiments of the invention.

Said drawing shows a platform of large dimensions, generally between 1,000 m² and one or more hectares, composed, according to one of the preferred embodiments, of a layer of clay (1) whereon a waterproof containment membrane is laid, such as for example a film of polyethylene (2), which envelops the entire lower part of the system.

The membrane is protected above by a layer of sand (3) and by a layer of cement (4) so as to create a large resistant and waterproof square on which to install the system.

The cement base is slightly tilted towards the point of collection of the percolate, composed of a watertight well (5).

A comb collector (6) is placed on the cement base (4) for the collection of the percolate and biogas, made up of a series of porous pipes intercalated between the straw bales (7) and protected by sand or gravel (8).

The comb collector (6) is able to transfer by channelling both percolate and biogas to the well (5) where gas and liquid are separated.

The substantially dry cellulose waste, with such a water content as to avoid spontaneous digestion, recovered at the time of collection, is preferably collected in bales due to the ease of transport and the improved structural features and is accumulated on the cement square and above the pipes to form a pile (9).

The bales are arranged one on the other in an ordered manner to form a structure with slanting walls up to a height of 20 m and preferably around 10 m.

During the formation of the pile a series of flat surfaces is created so as to form an extremely porous and permeable structure, the bales having a degree of vacuum of around 0.8 and density of 200 kg/m³.

This pile, being formed by dry materials, cannot undergo aerobic or anaerobic digestion with dispersion of biogas or odours or risk of fire due to self-combustion. When the pile is completed the biogas extraction wells (5) and the pipe for recycling the percolate (11) are inserted and, on the top of the pile, a comb distributor of the percolate (12) and grille collector for aspiration of the biogas (13), connected on one side to a biogas collector (14) and on the other to the atmosphere via a valve (15).

This enables, by opening the valve (15) and closing a cut-off valve (16) placed on the collector (14), controlled aspiration of the air in the pile.

In the final phase the pile is covered with a polyethylene membrane (17) which is sealed by welds (18) to the lower containment sheet (2) so as to form an airtight envelope impermeable to infiltrations of air.

As already mentioned, the possibility however remains to allow air to enter the percolate plenum by actuating the valves (15) and (16).

The piled dry material gives rise to very slow aerobic digestion, however if wetted in a short time lively aerobic digestion takes place with air and anaerobic digestion without air.

Wetting is carried out by feeding the percolate well (5) with water, liquid manure or percolates of other digestion processes (20) in order to accelerate the process and by actuating a recirculation pump (19).

The biogas or air is aspirated by a fan (21).

It should be noted at this point that other set-ups of the bioreactor are possible and whereof Fig. 1 represents a preferred set-up. For example it is possible to place a plenum on the bottom floor rather than the comb of pipes for aspiration of the biogas and collection of the percolate and with an appropriate arrangement of the valves and of the fan reverse the flow of air and/or biogas without altering the inventive concept.

The large size and simplicity of building of the bioreactor and the method used for its operation as described herein below are important.

According to the present invention it has been seen that it is advantageous, after wetting, to perform aerobic digestion for a period of 1 or 2 days by actuating the fan (21) and allowing the ambient air to bleed from the valve (15) which is completely open, while the valve (16) is closed.

Aerobic digestion compared to anaerobic digestion is in fact much faster and has good exothermy and therefore enables the mass to be heated to the required temperature around 35°C, optimal for the anaerobic mesophilic process.

The large volumes, low exchange surface and weak exothermy of the anaerobic digestion combine to maintain the temperature of the mass.

It is however possible to work always at an optimal temperature by generating heat in the mass by the input of a sufficient quantity of air for producing it by aerobic digestion.

This combined anaerobic/aerobic mode of operation is activated when the temperature of the biogas in the well (20) drops below 30°C and is achieved by regulating and modulating the valve for aspiration of the ambient air (15) and closing the valve (16) and measuring the flow rate of aspirated air.

The flow rate of aspirated air depends, in addition to the environmental conditions, on the size of the pile and is in general of a smaller order of magnitude than the flow rate of biogas.

It has been seen how it is advantageous to operate in such a way that the concentration of oxygen in the biogas is below 2% in volume and preferably around 0.1 % until the optimal temperature of 35°C is restored.

Having achieved this condition, pure anaerobic digestion is restored by closing the valve for aspiration of the air (15) and opening the valve (16).

It is particularly important to observe how aerobic digestion can take place simultaneously to anaerobic digestion, during operation, representing an innovative system of thermal regulation which does not use external energy sources, particularly not heat exchangers.

Heat transfer surfaces are not therefore necessary for maintaining the temperature but instead the exothermy generated by slow aerobic combustion of the straw is used.

Initial aerobic digestion has in particular a further advantage: it has in fact been found, surprisingly, that straw, after having undergone an aerobic treatment, has a much higher reactivity in the anaerobic treatment to the extent that it is possible to finish the process of production of biogas in times of the order of 1-2 years, times compatible therefore with the seasonal times of collection.

A further acceleration of the process is possible, having reached the optimal temperature, by adding slurry rich in cellulosic methanogenic bacteria to the pile.

It has been seen that in this way the optimal time for exploiting the bioreactor is around 14 months and during this period recirculation continues of the water that may have collected on the base (11) via the special pump (19) as suggested by the prior art and the biogas is extracted via the special fan (21) to send it for use, such as for example to an internal combustion engine for producing electrical energy.

During the entire anaerobic phase, also associated with the aerobic one only in relation to the need to maintain the optimal temperature, the bioreactor is airtight and with a slight vacuum and the membrane (17) adheres to the pile, compresses the mass and is supported by it.

Buildings or walls in concrete are not necessary for containing the reaction gas which remains in the empty space of the pile as in a large container.

Any surplus of biogas production is compensated by the high volume of the pile which also has the functions of a large tank.

At the end of the operation, after approximately 14 months of operation, access of the external air is gradually opened via the valve (15), closing the valve (16), and returning to aerobic digestion operation. The purpose of this is to eliminate the residual volatile substances and, by means of the reaction heat produced, evaporate part of the water and therefore biodrying the pile.

This operation is in practice one of composting which produces a stabilised fertiliser.

The operation lasts around ten days at the end of which the covering sheet is removed to enable the farm machinery to dismantle the pile.

The composted material, which contains all the nutritional elements removed from the soil, is transferred back to the straw removal fields, contributing to maintaining fertility, with the same means used for transport of the straw.

To optimise the costs three piles are therefore necessary: one for filling during collection, one for simultaneous emptying and one for operation.

The covering sheet can be cut into strips which can be reused in situ or shredded, washed in situ and sent for recycling of the polyethylene to produce new sheets.

The process can be summed up briefly as follows:
- formation of the self-extinguishing pile on the cement square, laying of the pipes, of the well, of the sheets and its waterproofing.
- Wetting and activation of the pile.
- Aerobic pre-treatment (bringing to temperature and hydrolysis).
- Addition of cellulosic enzymes and microorganisms.
- Anaerobic and combined aerobic/anaerobic treatment with recycling of the percolates.
- Final aerobic treatment.
- Recovery or recycling of the upper sheet.
- Recycling on agricultural soil of the nutritional elements contained in the residual composted material.

If only the main operations are described, we therefore have:
- a stage of anaerobic digestion which can be used to bring the mass to the required temperature. A stage which in particular climatic conditions may possibly be avoided;
- a stage of aerobic digestion, possibly integrated with a stage of aerobic digestion or partially aerobic digestion, associated with the aerobic one, aerobic oxidations being aimed at the production of heat for thermostatting of the mass at the required temperature of anaerobic digestion.

The main advantages of the process of the present invention are:
1) high production of biogas per quantity of biomass
2) recovery of organic and humic substances useful for the soil
3) reduction of gas emissions that affect climate
4) suitable decomposition time for continuously feeding energy production plants
5) elimination of gaseous emissions and polluting liquids
6) minimum consumption and total recovery of materials for building of the plant and therefore very low investment costs and low running costs.

These advantages and others can be obtained from the example of application reported herein below which describes work underway at the Applicant's premises. This example is therefore to be considered demonstrative and non-limiting.

### Example of application

The application example relates to the surface sown with rice currently available for a total of 1,000 hectares and production of straw of around 5,000 t/year with 15% humidity equal to a volume of 25,000 m³.

Referring to Fig. 1, the pile of straw bales has a square base of 57 metres per side, a surface of around 3,300 m² and a height of 12 metres. The size of the cement platform is to be around 60 x 60 = 3600 m².

Operation is to last for 14 months.

The pile is formed on the cement platform with sheath or containment sheet and, at the end of banking-up, covered with a sheet welded along the sides with the sheath/sheet of the base as indicated in Fig. 1.

After sealing wetting is performed (700 m3 water) and the aerobic phase launched so as to activate the hydrolysis phase and raise the temperature of the pile between 30 and 40°C.

Having reached the temperature, the strict anaerobic conditions are established. Once these are achieved, 50 m3 of slurry rich in cellulosic methanogenic bacteria are added to the pile.

In the case of a lowering of temperature at very cold times, air is allowed to aspirate to ensure a sufficient percentage of aerobic operation in order to maintain the optimal temperature. The estimated biogas production is 1,800,000 - 2,000,000 Nm3/year with a methane content > 50%, therefore with a production of methane of 980,000 - 1,100,000 Nm3/year (745 - 836 t/year) equal to 112 - 126 Nm3/hour of methane. The biogas obtained in this way is fed to an internal combustion engine with alternator to produce a power equal to 370 - 420 kW of electrical energy.

At the end of the degradation process, after the final aerobic phases that will remove 20% of residual water, 3,000 t of soil will remain with 65% of dry substance to be spread over the land with the normal muckspreading machines.

After 12 months of operation, while the exhausted pile still has 2 months of life, a new collection is performed and a new pile built which is activated shortly before the start of banking-up of the old pile.

At this point it should noted that with an occupied surface of 3,600 m² there is installed power of around 400 kW while if there were material for covering a hectare of land the power would be 1.1 MW, equal to around 11 million kWh of energy produced.

The surface occupied by the bioreactor appears high but, if compared with other energies from renewable sources, it appears minimal. For example a hectare of land with solar panels would give a power of 1.2 MW limited to 8 hours of sun and therefore annual energy production of only 3.6 million kWh and with enormously higher investments.

If it were possible to use the straw of 210,000 hectares sown with rice in Piedmont and Lombardy, there would be available electrical power of 84 MW and all the inorganic nutritional elements removed from the rice would be recovered and returned to the fields as soil, not including those contained in the grain. Bearing in mind that rice straw is a practically unused by-product, unlike, for example, corn straw, it has instead an environmental cost, and that the costs, including energy costs, of investment and management are very low, the net energy produced in the process described can be considered as the most economical one which can currently be obtained from agricultural biomasses.

The bioreactor which forms the object of the present invention represents, due to the low cost, working simplicity, energy yield net of the energy for investment and operation, recovery and reuse in the soils of nutrient substances, a major opportunity which is not just environmental and for agriculture and for the farms which house it but also a means of meeting with renewable energies part of the regional and national energy requirement.

### BIBLIOGRAPHY

Holzapfel-Pschorn A, Conrad R, Seiler W. (1986) Effects of vegetation on the emission of methane from submerged paddy soil. Plant Soil. 92:223.
Jenkins BM (1996) Atmospheric pollutant emission factors from open burning of agricultural and forest biomass by wind tunnel simulations. Final report (3 vols.), CARB Project A932-126. Sacramento, California***.***
Bokus WW, Webster RK, Wick CM, Jackson LF (1979) Rice residue disposal influences overwintering inoculum level of Sclerotium oryzae and stem rot severity. Phytopathology 69 (8): 862-865*.*
Barlaz MA, Ham RK, Schaefer DM (1990) Methane production from municipal refuse: a review of enhancement techniques and microbial dynamics. Crti. Rev. In Environ. Control 19 (6): 557***.***
Kadam K.L., Forrest L.H., Jacobson L.W. (2000) Rice straw as a lignocellulosic resource: collection, processing, transportation and environmental aspects. Biomass and bioenergy, 18, 369***.***

## Claims

1. Process for the biological treatment of dry cereal straw, in particular rice straw containing cellulose and recycling on agricultural land of the residual composted material, comprising the following phases:
- formation of a pile (9) of said rice straw collected in bales (7) arranged one on the other in an ordered manner to form a structure with slanting walls on a platform disposed on the soil;
- insertion in the pile of biogas extraction wells (5) and a pipe (11) for recycling the percolate and, on the top of the pile, a comb distributor (12) of the percolate and grille collector (13) for aspiration of the biogas, connected on one side to a biogas collector (14) and on the other to the atmosphere via a valve (15);
- waterproofing of the pile sealing by welds a membrane (17) covering the pile to a lower containment membrane (2); this pile, being composed of dry materials, cannot undergo aerobic or anaerobic digestion with dispersion of biogas or odours or risk of fire through self-combustion;
- wetting and activation of the pile;
- aerobic pre-treatment for the bringing to temperature and hydrolysis; anaerobic treatment with recycling of percolates;
- maintaining of the temperature by controlled feeding of air and therefore with exothermic aerobic digestion regulated perfectly by means of the quantity of air fed, through regulation and modulation of said a valve (15) for the aspiration of ambient air and closure of a valve (16) for the aspiration of biogas from the pile (9);
- final aerobic treatment to evaporate the excess of water
- recycling on agricultural land of the residual composted material containing all the nutritional elements removed from the soil.

2. Process according to claim 1, wherein the pile is wetted with water, liquid manure or percolates of other digestion processes.

3. Process according to claim 1 or 2, wherein transfer is provided of the percolate and biogas from the base of the pile (9) to at least one hermetically sealed well (5) where gas and liquid are separated.

4. Process according to any one of the previous claims, wherein recycling is provided of the percolate with distribution at the top of the pile (9).

5. Process according to any one of the previous claims, wherein at the top of the pile aspiration is provided of biogas or the blowing of air.

6. Process according to claim 1, wherein after said phase of aerobic pre-treatment the addition is provided of slurry rich in cellulosic methanogenic bacteria.

7. Process according to any one of the previous claims, wherein the biogas produced is sent for use, for example to an internal combustion engine to produce electrical energy.

8. System for the treatment of the straw of cereals, in particular rice straw containing cellulose and recycling on agricultural land of the residual composted material, comprising:
- a waterproof platform on the soil suitable for holding a pile (9) of said rice straw collected in bales (7) arranged one on the other in an ordered manner to form a structure with slanting walls, said platform being formed by a layer of clay 1, whereon a waterproof containment membrane (2), a layer of sand (3) and a layer of cement (4) are placed;
- means placed on said platform for collecting percolate and biogas produced through digestion of said cellulose material, comprising a comb collector (6) formed by a series of porous pipes intercalated between the bales (7) and protected by sand or gravel (8), placed at a slight slant on said platform and leading into a watertight well (5), where separation between the gas and liquid takes place;
- means for recycling the percolate and its distribution at the top of the pile (9);
- means for aspirating biogas from the top of the pile or for blowing air therein, comprising an aspiration fan (21) and a grille collector (13) for aspiration of the biogas, connected on one side to a collector (14), whereon a cut-off valve (16) is placed and on the other side to the atmosphere via a valve (15), in such a way that by opening the valve (16) and closing the valve (15) the biogas is aspirated from the pile (9) and, vice versa, by closing the valve (16) and opening the valve (15) air is blown into the pile;
- means for waterproofing the entire system consisting in a covering polyethylene membrane (17) which is sealed to said lower containment membrane (2) by welds (18).

9. System according to claim 8, wherein said means of recycling and distribution of the percolate comprise a pump (19) for aspiration from said well (5), a recycling pipe (11) and a comb distributor (12).

10. System according to claim 8 or 9, wherein means are provided for feeding said well of the percolate (5) with water, liquid manure of percolates of other digestion processes (20), for later recirculation in the pile (9) by means of the pump (8).

## Patentansprüche

1. Verfahren zur biologischen Behandlung von getrocknetem Getreidestroh, insbesondere zellulosehaltigem Reisstroh, und zur Rückführung des verbleibenden, kompostierten Materials auf landwirtschaftliches Gebiet, umfassend die folgenden Phasen:
- Bildung eines Stapels (9) des in Ballen (7) gesammelten Reisstrohs, welche einer auf dem anderen in einer geordneten Weise angeordnet sind, unter Bildung einer Struktur mit abgeschrägten Wänden auf einer auf dem Boden angeordneten Plattform;
- Einführen von Biogasextraktionsgefäßen (5) und eines Rohrs (11) zur Rückführung des Perkolats in den Stapel, und, ganz oben auf dem Stapel, eines Rechenverteilers (12) für das Perkolat und eines Gitterkollektors (13) zur Absaugung des Biogases, auf der einen Seite mit einem Biogaskollektor (14) verbunden, und auf der anderen Seite zur Atmosphäre über ein Ventil (15) verbunden;
- Wasserabdichten des Stapels durch Verschließen einer Membran (17), welche den Stapel abdeckt, mittels Verschweißungen zu einer Membran eines unteren Behälters (2); wobei dieser Stapel, welcher aus trockenen Materialien besteht, keine aerobe oder anaerobe Fermentation mit Dispersion von Biogas oder Dämpfen oder Feuergefahr durch selbständige Verbrennung erfahren kann;
- Befeuchten und Aktivieren des Stapels;
- aerobe Vorbehandlung zur Erreichung von Temperatur und Hydrolyse; anaerobe Behandlung mit Rückführung von Perkolaten;
- Aufrechterhalten der Temperatur durch kontrollierte Zufuhr von Luft und daher unter exothermer aerober Fermentation, die vollkommen mittels der Menge zugespeister Luft reguliert wird, durch Regulierung und Veränderung des Ventils (15) zur Absaugung von Umgebungsluft, und Schließen eines Ventils (16) zur Absaugung von Biogas aus dem Stapel (9);
- finale aerobe Behandlung zur Verdampfung des Überschusses von Wasser
- Rückführung des verbleibenden kompostierten Materials, welches alle aus dem Boden entfernten Nährelemente enthält, auf landwirtschaftliches Gebiet.

2. Verfahren gemäß Anspruch 1, wobei der Stapel mit Wasser, Flüssigdung oder Perkolaten anderer Fermentationsprozesse befeuchtet wird.

3. Verfahren gemäß Anspruch 1 oder 2, wobei ein Transfer des Perkolats und Biogases vom Boden des Stapels (9) zu wenigstens einem hermetisch abgeschlossenen Gefäß (5), wo Gas und Flüssigkeit getrennt werden, bereitgestellt wird.

4. Verfahren gemäß einem der vorangehenden Ansprüche, wobei die Rückführung des Perkolats mit Verteilung ganz oben auf dem Stapel (9) bereitgestellt wird.

5. Verfahren gemäß einem der vorangehenden Ansprüche, wobei eine Absaugung von Biogas oder ein Einblasen von Luft ganz oben auf dem Stapel bereitgestellt wird.

6. Verfahren gemäß Anspruch 1, wobei nach der Phase der aeroben Vorbehandlung die Zugabe einer Aufschlämmung, die reich an methanogenen Zellulosebakterien ist, bereitgestellt wird.

7. Verfahren gemäß einem der vorangehenden Ansprüche, wobei das produzierte Biogas zur Verwendung, beispielsweise zu einem Verbrennungsmotor zur Produktion elektrischer Energie, geleitet wird.

8. System zur Behandlung von Getreidestroh, insbesondere zellulosehaltigem Reisstroh, und Rückführung des verbleibenden kompostierten Materials auf landwirtschaftliches Gebiet, umfassend:
- eine wasserfeste Plattform auf dem Boden, die geeignet ist zur Aufnahme eines Stapels (9) von in Ballen (7) gesammelten Reisstroh, wobei einer auf dem anderen in einer geordneten Weise unter Bildung einer Struktur mit abgeschrägten Wänden angeordnet ist, wobei die Plattform durch eine Schicht Ton 1 gebildet wird, worauf eine wasserfeste Behältermembran (2), eine Schicht Sand (3) und eine Schicht Zement (4) platziert werden;
- auf der Plattform platzierte Mittel zur Aufnahme von Perkolat und Biogas, hergestellt durch Fermentation des Zellulosematerials, umfassend einen Rechenkollektor (6), gebildet durch eine Serie von porösen Rohren, die zwischen die Ballen (7) eingeschoben und durch Sand oder Kies (8) geschützt werden, platziert bei einer leichten Abschrägung der Plattform und in ein wasserdichtes Gefäß (5) führend, wo die Trennung zwischen dem Gas und der Flüssigkeit stattfindet;
- Mittel zur Rückführung des Perkolats und dessen Verteilung ganz oben auf dem Stapel (9);
- Mittel zur Absaugung von Biogas ganz oben auf dem Stapel oder zum Einblasen von Luft in diesen, umfassend einen Absauglüfter (21) und einen Gitterkollektor (13) zur Absaugung des Biogases, auf einer Seite verbunden mit einem Kollektor (14), worauf ein Abschaltventil (16) platziert ist, und auf der anderen Seite mit der Atmosphäre über ein Ventil (15), in einer solchen Weise, dass durch Öffnen des Ventils (16) und Schließen des Ventils (15) das Biogas aus dem Stapel (9) abgesaugt wird, und, umgekehrt, durch Schließen des Ventils (16) und Öffnen des Ventils (15) Luft in den Stapel geblasen wird;
- Mittel zur Wasserabdichtung des gesamten Systems, bestehend aus einer Polyethylen-Abdeckmembran (17), welche mit der unteren Behältermembran (2) über Verschweißungen (18) versiegelt ist.

9. System gemäß Anspruch 8, wobei die Mittel zur Rückführung und Verteilung des Perkolats eine Pumpe (19) zur Absaugung aus dem Behälter (5), ein Rückführungsrohr (11) und einen Rechenverteiler (12) umfassen.

10. System gemäß Anspruch 8 oder 9, wobei Mittel zur Beschickung des Gefäßes des Perkolats (5) mit Wasser, Flüssigdung von Perkolaten aus anderen Fermentationsprozessen (20) für die spätere Rezirkulation in dem Stapel (9) mit der Pumpe (8) bereitgestellt werden.

## Revendications

1. Procédé pour le traitement biologique de paille céréalière sèche, en particulier de paille de riz contenant de la cellulose et le recyclage sur une terre agricole de la matière compostée résiduelle, comprenant les phases suivantes :
- formation d'une pile (9) de ladite paille de riz rassemblée en balles (7) agencées les unes sur les autres d'une manière ordonnée afin de former une structure avec des parois inclinées sur une plate-forme disposée sur le sol ;
- insertion dans la pile de puits d'extraction de biogaz (5) et d'un tuyau (11) pour recycler le percolat et, sur le sommet de la pile, d'un distributeur en peigne (12) du percolat et d'un collecteur grillagé (13) pour l'aspiration du biogaz, raccordé sur un côté à un collecteur de biogaz (14) et sur l'autre à l'atmosphère par l'intermédiaire d'une valve (15) ;
- imperméabilisation de la pile par scellage par des soudures d'une membrane (17) recouvrant la pile sur une membrane de confinement inférieure (2) ; cette pile, qui est composée de matières sèches, ne peut pas subir de digestion aérobie ou anaérobie avec dispersion de biogaz ou d'odeurs ou risque d'incendie par combustion spontanée ;
- humidification et activation de la pile ;
- prétraitement aérobie pour la mise à la température et l'hydrolyse ; traitement anaérobie avec recyclage de percolats ;
- maintien de la température par introduction contrôlée d'air et par conséquent avec digestion aérobie exothermique parfaitement régulée au moyen de la quantité d'air introduite, par régulation et modulation de ladite valve (15) pour l'aspiration d'air ambiant et fermeture d'une valve (16) pour l'aspiration de biogaz à partir de la pile (9) ;
- traitement aérobie final afin d'évaporer l'excès d'eau
- recyclage sur une terre agricole de la matière compostée résiduelle contenant tous les éléments nutritionnels retirés du sol.

2. Procédé selon la revendication 1, dans lequel la pile est humidifiée avec de l'eau, du lisier liquide ou des percolats d'autres processus de digestion.

3. Procédé selon la revendication 1 ou la revendication 2, dans lequel il est fourni un transfert du percolat et du biogaz de la base de la pile (9) à au moins un puits scellé hermétiquement (5) où le gaz et le liquide sont séparés.

4. Procédé selon l'une quelconque des revendications précédentes, dans lequel il est fourni un recyclage du percolat avec distribution au sommet de la pile (9).

5. Procédé selon l'une quelconque des revendications précédentes, dans lequel au sommet de la pile il est fourni une aspiration de biogaz ou le soufflage d'air.

6. Procédé selon la revendication 1, dans lequel après ladite phase de prétraitement aérobie il est fourni l'ajout de boue riche en bactéries méthanogènes cellulosiques.

7. Procédé selon l'une quelconque des revendications précédentes, dans lequel le biogaz produit est envoyé en vue d'une utilisation, par exemple vers un moteur à combustion interne afin de produire de l'énergie électrique.

8. Système pour le traitement de la paille de céréales, en particulier de paille de riz contenant de la cellulose et le recyclage sur une terre agricole de la matière compostée résiduelle, comprenant :
- une plate-forme imperméable sur le sol convenant pour maintenir une pile (9) de ladite paille de riz rassemblée en balles (7) agencées les unes sur les autres d'une manière ordonnée afin de former une structure avec des parois inclinées, ladite plate-forme étant formée par une couche d'argile 1, sur laquelle une membrane de confinement imperméable (2), une couche de sable (3) et une couche de ciment (4) sont placées ;
- des moyens placés sur ladite plate-forme pour recueillir le percolat et le biogaz produits par digestion de ladite matière cellulosique, comprenant un collecteur en peigne (6) formé par une série de tuyaux poreux intercalés entre les balles (7) et protégés par du sable ou du gravier (8), placé selon une légère inclinaison sur ladite plate-forme et menant jusque dans un puits étanche (5), où la séparation entre le gaz et le liquide a lieu ;
- des moyens pour recycler le percolat et sa distribution au sommet de la pile (9) ;
- des moyens pour aspirer du biogaz à partir du sommet de la pile ou pour souffler de l'air en son sein, comprenant un ventilateur d'aspiration (21) et un collecteur grillagé (13) pour l'aspiration du biogaz, raccordé sur un côté à un collecteur (14), sur lequel une valve d'arrêt (16) est placée et sur l'autre côté à l'atmosphère par l'intermédiaire d'une valve (15), de telle manière qu'en ouvrant la valve (16) et en fermant la valve (15) le biogaz est aspiré à partir de la pile (9) et, vice versa, en fermant la valve (16) et en ouvrant la valve (15) de l'air est soufflé à l'intérieur de la pile ;
- des moyens pour imperméabiliser le système entier consistant en une membrane de polyéthylène couvrante (17) qui est scellée sur ladite membrane de confinement inférieure (2) par des soudures (18).

9. Système selon la revendication 8, dans lequel lesdits moyens de recyclage et de distribution du percolat comprennent une pompe (19) pour l'aspiration à partir dudit puits (5), un tuyau de recyclage (11) et un distributeur en peigne (12).

10. Système selon la revendication 8 ou la revendication 9, dans lequel des moyens sont fournis pour alimenter ledit puits du percolat (5) en eau, en lisier liquide de percolats d'autres processus de digestion (20), pour une recirculation ultérieure dans la pile (9) au moyen de la pompe (8).
